# EUROPEAN PATENT APPLICATION

(11) **EP 4 197 572 A1**
(43) Date of publication of application: **21.06.2023**
(21) Application number: 21306832.3
(22) Date of filing: 17.12.2021
(51) Int. Cl.: A61M 5/142, A61M 5/315

(54) **THERMOELECTRIC GENERATOR FOR POWERING AUTOINJECTOR**

(71) Applicant: Becton, Dickinson and Company, Franklin Lakes, NJ 07417-1880 (US)
(72) Inventor: CHARPENTIER, Marion, 38600 Fontaine (FR)
(74) Representative: Germain Maureau

(57) **Abstract**

Provided herein is a wearable drug delivery device including a housing having a skin-facing surface defining a cannula opening, and a viewing window, a container disposed within the housing and configured to store the pharmaceutical composition therein, a cannula in fluid communication with the container, the cannula transitionable from an initial position in which the cannula is disposed within the housing to a use position in which the cannula extends through the cannula opening, a visual identifier aligned with a portion of the viewing window, wherein a position of the visual identifier relative to the viewing window is dependent on a volume of the pharmaceutical composition within the container, a thermoelectric generator configured to supply power to the drug delivery device, wherein the housing is configured to be releasably attached to a patient's skin.

## Description

### BACKGROUND OF THE INVENTION

### Field of the Invention

The present disclosure relates to wearable drug delivery devices with one or more components powered by thermoelectric generators.

### Description of Related Art

Wearable medical devices, such as drug delivery devices, have the benefit of providing therapy to the patient at a location remote from a clinical facility and/or while being worn discretely under the patient's clothing. The wearable medical device can be applied to the patient's skin and configured to automatically deliver a dose of a pharmaceutical composition within a predetermined time period after applying the wearable medical device to the patient's skin, such as after a 27 hour delay. After the device delivers the pharmaceutical composition to the patient, the patient may subsequently remove and dispose of the device.

Such wearable devices are typically powered by one or more batteries. For example, such devices may include an electric motor for expelling the pharmaceutical composition. In addition, increasingly such wearable devices are equipped with "smart" features, such as indicators (e.g., light emitting diodes, LEDs), communication devices (e.g., BLUETOOTH modules), and like functionality. The use of batteries in such applications raises concerns of increased waste. Accordingly, there is a need in the field for a sustainable energy source that can be used with a wearable drug delivery device and has the potential for being reusable.

### SUMMARY OF THE INVENTION

Provided herein is a wearable drug delivery device including a housing having a skin-facing surface defining a cannula opening, and a viewing window, a container disposed within the housing and configured to store the pharmaceutical composition therein, a cannula in fluid communication with the container, the cannula transitionable from an initial position in which the cannula is disposed within the housing to a use position in which the cannula extends through the cannula opening, a visual identifier aligned with a portion of the viewing window, wherein a position of the visual identifier relative to the viewing window is dependent on a volume of the pharmaceutical composition within the container, a thermoelectric generator configured to supply power to the drug delivery device, wherein the housing is configured to be releasably attached to a patient's skin.

### BRIEF DESCRIPTION OF THE DRAWINGS

Figure 1 is a perspective view of a drug delivery device according to non-limiting embodiments or aspects described herein;
Figure 2 is a perspective view of a drug delivery device according to non-limiting embodiments or aspects described herein;
Figure 3 is a schematic view of a drug delivery device according to non-limiting embodiments or aspects described herein;
Figure 4 is a perspective view of a drug delivery device according to non-limiting embodiments or aspects described herein;
Figure 5 is a perspective view of a drug delivery device according to non-limiting embodiments or aspects described herein;
Figure 6 is a top view of a drug delivery device according to non-limiting embodiments or aspects described herein;
Figure 7 is a top view of a drug delivery device according to non-limiting embodiments or aspects described herein; and
Figure 8 is a schematic view of a thermoelectric generator for use with a drug delivery device according to non-limiting embodiments or aspects described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

All numbers used in the specification and claims are to be understood as being modified in all instances by the term "about". By "about" is meant a range of plus or minus ten percent of the stated value. As used in the specification and the claims, the singular form of "a", "an", and "the" include plural referents unless the context clearly dictates otherwise. The terms "first", "second", and the like are not intended to refer to any particular order or chronology, but instead refer to different conditions, properties, or elements. By "at least" is meant "greater than or equal to".

As used herein, the term "cannula" may refer to needles, cannulae, catheters, and like structures that allow for fluid communication between a pharmaceutical composition container and a patient.

As used herein, the term patient may refer to a human or non-human animal.

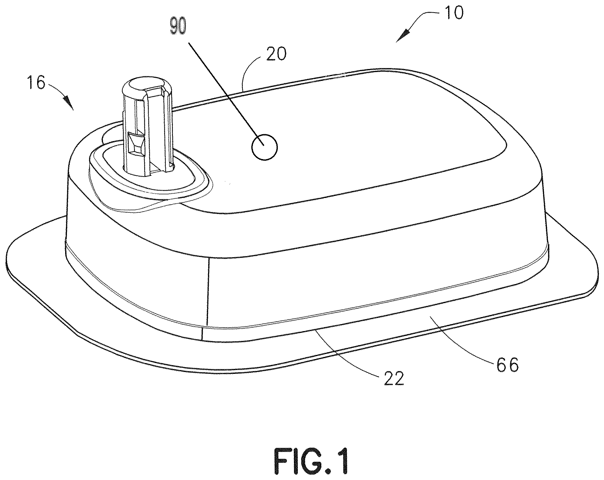

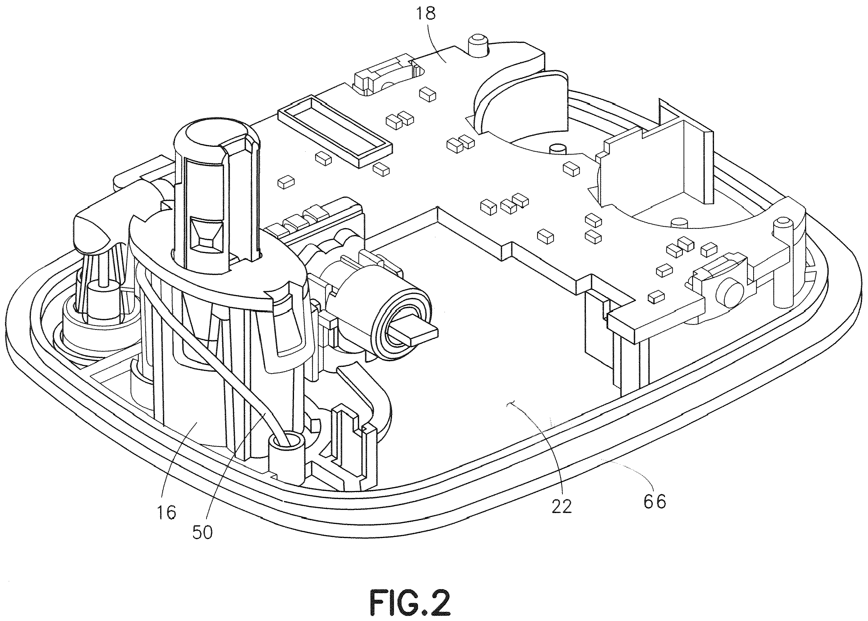

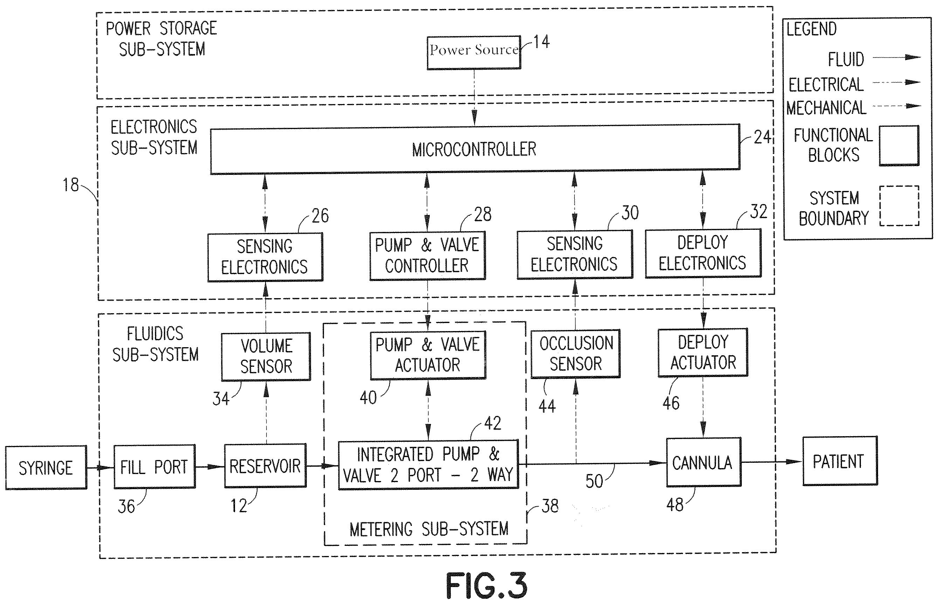

Referring to **FIGS. 1-3****,** provided herein is a drug delivery device 10. Drug delivery device 10 may include a container 12, a power source 14, an insertion mechanism 16, control electronics 18, a cover 20, and/or a base 22. In non-limiting embodiments or aspects, the drug delivery device 10 is a wearable automatic injector, such as an insulin or bone marrow stimulant delivery device. The drug delivery device 10 may be mounted onto the skin of a patient and triggered to inject a pharmaceutical composition from the container 12 into the patient. The drug delivery device 10 may be pre-filled with the pharmaceutical composition, or it may be filled with the pharmaceutical composition by the patient or medical professional prior to use.

The drug delivery device 10 is configured to deliver a dose of a pharmaceutical composition, e.g., any desired pharmaceutical composition, into the patient's body by a subcutaneous injection at a slow, controlled injection rate, for example through use of a motor (e.g., an electric motor) or pump to cause pharmaceutical composition to be expelled from container 12, for example by compressing one or more areas of container 12, by causing a stopper to be moved within container 12 to lessen the volume of container 12, or other like mechanisms known to those of skill in the art. Exemplary time durations for the delivery achieved by the drug delivery device 10 may range from about 5 minutes to about 60 minutes, but are not limited to this exemplary range. Exemplary volumes of the pharmaceutical composition delivered by the drug delivery device 10 may range from about 0.1 milliliters to about 10 milliliters, but are not limited to this exemplary range. The volume of the pharmaceutical composition delivered to the patient may be adjusted.

Referring again to **FIGS. 1-3****,** in one aspect or embodiment, the power source 14 is a thermoelectric generator 66, and thermoelectric generator 66 may provide power to other components, for example a pump, motor, control electronics 18, indicators, and/or fluidics components. The control electronics 18 may include a microcontroller 24, sensing electronics 26, a pump/motor and valve controller 28, sensing electronics 30, and deployments electronics 32, which control the actuation of the drug delivery device 10. The drug delivery device 10 may also include a fluidics sub-system that includes the container 12, volume sensor 34 for the container 12, a container fill port 36, and a metering system 38 including a pump and valve actuator 40 and a pump and valve mechanism 42. The fluidic sub-system may further include an occlusion sensor 44, a deploy actuator 46, a cannula 48 for insertion into a patient's skin, and a fluid line 50 in fluid communication with the container 12 and the cannula 48. In an alternative aspect or embodiment, the occlusion sensor is a system for measuring the motor current. In one aspect or embodiment, the insertion mechanism 16 is configured to move the cannula 48 from a retracted position positioned entirely within the device 10 to an extended position where the cannula 48 extends outside of the device 10. The drug delivery device 10 may operate in the same manner as discussed in U.S. Patent No. 10,449,292, the content of which is incorporated herein by reference in its entirety.

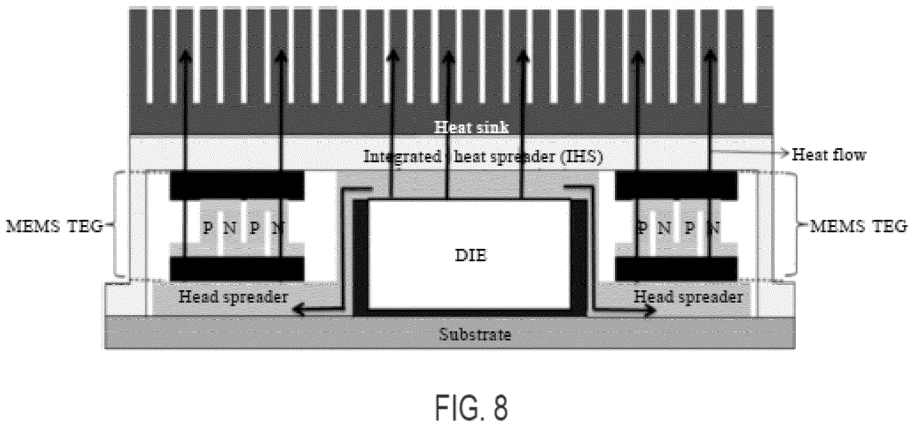

With further reference to thermoelectric generator 66, those of skill will appreciate that multiple arrangements of thermoelectric generators are known, as described in Jaziri et al. A Comprehensive Review of Thermoelectric Generators: Technologies and Common Applications. Energy Reports 2020, Vol. 6, pp. 264-287, the content of which is incorporated herein by reference in its entirety. Such thermoelectric generators, for those depicted schematically in **FIG. 8**, operate through the Seebeck effect, in which a temperature difference is converted to electric voltage through a thermocouple. In non-limiting embodiments or aspects, thermoelectric generator 66 is arranged on the patient's skin. In non-limiting embodiments or aspects, the temperature gradient exists between the patient's skin and ambient air and/or the interior of drug delivery device 10. In non-limiting embodiments or aspects, thermoelectric generator 66 is arranged between base 22 of drug delivery device 10 and the patient's skin.

Thermoelectric generators 66 as described herein may include a plurality of thermocouples, heat spreaders, and/or heat sinks. Suitable thermocouples may be formed of any suitable materials known to those of skill in the art, including, without limitation, polymers and metals. In non-limiting embodiments or aspects, one or more of the thermocouples are formed of poly(3,4-ethylenedioxythiophene) polystyrene sulfonate and/or nickel ethenetetrathiolate. In non-limiting embodiments or aspects, one or more of the thermocouples are formed of silicon, gold, silver, nickel, palladium, copper, antinomy, tellurium, bismuth, selenium, and/or mixtures and alloys thereof. In non-limiting embodiments or aspects, the thermocouples are Type N thermocouples, formed of alloys of nickel and silicon (e.g., alloys of nickel, chromium, silicon, and magnesium). In non-limiting embodiments or aspects, the thermocouples are Type P thermocouples, formed of alloys of palladium (e.g., alloys of palladium, platinum, and gold). In non-limiting embodiments or aspects, one or more different types of thermocouples are included in the thermoelectric generator 66. For example, as shown in the non-limiting embodiment of **FIG. 8**, a micro-electromechanical system (MEMS) thermoelectric generator includes a plurality of Type N and Type P thermocouples. Thermoelectric generator 66 may also include one or more heat spreaders to increase the efficiency of the conversion of temperature gradient to electric voltage.

The thermocouples used in the thermoelectric generator 66 may be provided on a substrate. Suitable substrates may be formed of any suitable materials known to those of skill in the art, including without limitation silicon, alumina, polyimide, cellulose, ceramic, fabric, and/or paper. Those of skill in the art will appreciate that for use with wearable drug delivery devices, flexible substrates, such as those formed of paper and/or fabric, may provide superior comfort and performance.

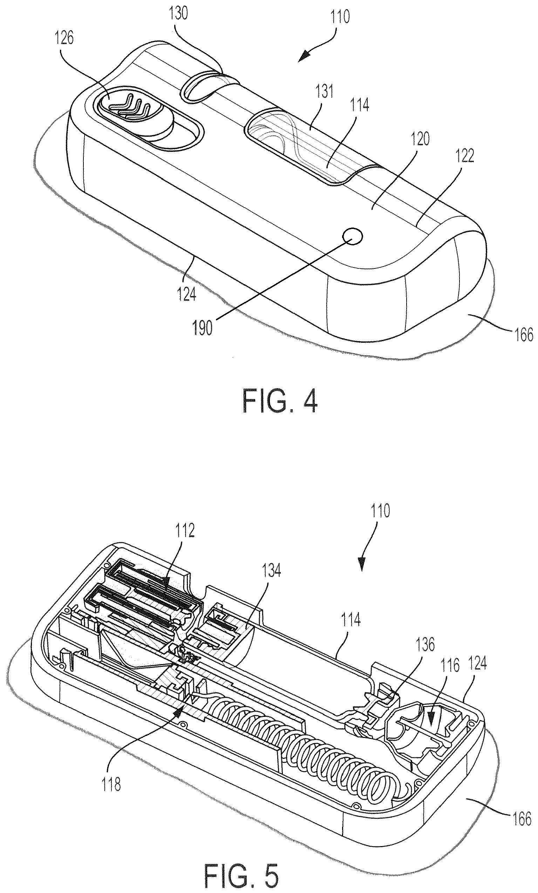

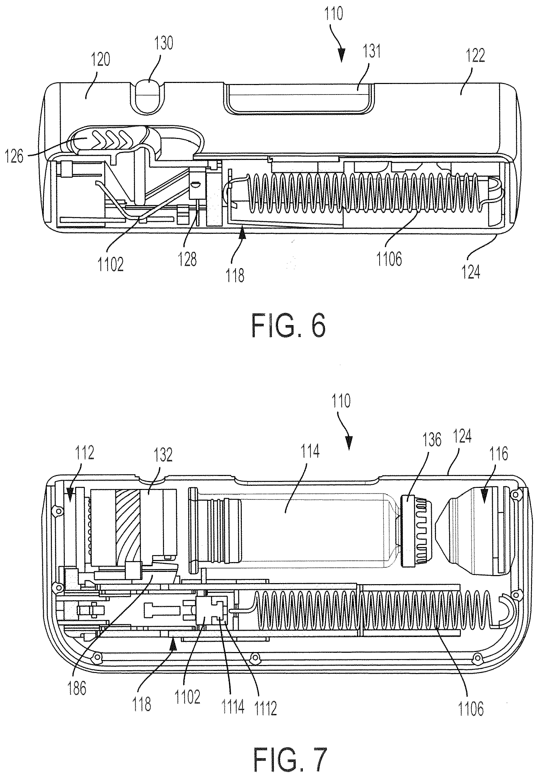

With reference to **FIGS. 4-7****,** shown is another non-limiting embodiment or aspect of a drug delivery device 110. Drug delivery device 110 may be used to deliver any desired pharmaceutical composition into the patient's body by a subcutaneous injection at a slow, controlled injection rate. Exemplary time durations for the delivery achieved by the delivery device 110 may range from about 5 minutes to about 60 minutes, but are not limited to this exemplary range. Exemplary volumes of the pharmaceutical composition delivered by the delivery device 110 may range from about 0.1 milliliters to about 10 milliliters, but are not limited to this exemplary range. The volume of the pharmaceutical composition delivered to the patient may be adjusted.

Referring to **FIGS. 4-7**, drug delivery device 110 according to one aspect of the present invention may include a drive assembly 112, a container 114 having a pierceable closure 136, a valve assembly 116, and a cannula actuator assembly 118. The drive assembly 112, the container 114, the valve assembly 116, and the cannula actuator assembly 118 are at least partially positioned within a housing 120. The container 114 and valve assembly 116 may be the container 114 and valve assembly 116 shown and described in International Publication No. WO 2015/081337, which is hereby incorporated by reference in its entirety. Cannula actuator assembly 118 may include a cannula actuator body having a cannula shuttle 1102, and the cannula 128 received by the cannula shuttle 1102 and configured to be in fluid communication with the container 114 as discussed above. The cannula actuator body, and thus cannula shuttle 1102, is biased from the pre-use position to the post-use position via an extension spring 1106, although other suitable biasing arrangements may be utilized. The cannula actuator body is released and free to move from the pre-use position to the use position upon engagement of the actuator button 126.

The housing 120 may include a top portion 122 and a bottom portion 124, although other suitable arrangements for the housing 120 may be utilized. In one aspect, the drug delivery system 110 is an injector device configured to be worn or secured to a user and to deliver a predetermined dose of a pharmaceutical composition provided within the container 114 via injection into the user. The system 110 may be utilized to deliver a "bolus injection" where a pharmaceutical composition is delivered within a set time period. The pharmaceutical composition may be delivered over a time period of up to 45 minutes, although other suitable injection amounts and durations may be utilized. A bolus administration or delivery can be carried out with rate controlling or have no specific rate controlling. The drug delivery device 110 may deliver the pharmaceutical composition at a fixed pressure to the user with the rate being variable. The general operation of the drug delivery device 110 is described and shown in International Publication Nos. WO 2013/155153 and WO 2014/179774, which are hereby incorporated by reference in their entirety. However, briefly, in a pre-use position of the drug delivery device 110, the container 114 is spaced from the drive assembly 112 and the valve assembly 116, and the cannula 128 is in a retracted position.

During the initial actuation of the drug delivery device 110, the drive assembly 112 engages the container 114 to move the container 114 toward the valve assembly 116, which is configured to pierce closure 136 of the container 114 and place the pharmaceutical composition within the container 114 in fluid communication with the cannula 128 via a tube (not shown) or other suitable arrangement. The drive assembly 112 is configured to engage a stopper of the container 114, which will initially move the entire container 114 into engagement with the valve assembly 116 due to the incompressibility of the fluid or pharmaceutical composition within the container 114. Thereafter, the stopper may be displaced under force from the drive assembly 112, thereby expelling the pharmaceutical composition from the container 114 through cannula 128. The initial actuation of the drug delivery device 110 is caused by engagement of the actuation button 126 by a user, which releases the cannula actuator assembly 118 and the drive assembly 12 as discussed below in more detail. During the initial actuation, the cannula 128 is still in the retracted position and about to move to the extended position to inject the user of the drug delivery device 110. When injection of the pharmaceutical composition is complete, cannula 128 is withdrawn.

The housing 120 of the drug delivery device 110 may include an indicator window 130 for viewing an indicator arrangement 132 configured to provide an indication to a user on the status of the drug delivery device 110 and a container window 131 for viewing the container 114. The indicator window 130 may be a magnifying lens for providing a clear view of the indicator arrangement 132. The indicator arrangement 132 may move along with the cannula actuator assembly 118 during use of the drug delivery device 110 to indicate a pre-use status, use status, and post-use status of the drug delivery device 110. The indicator arrangement 132 may provide visual indicia regarding the status, although other suitable indicia, such an auditory or tactile, may be provided as an alternative or additional indicia.

Drug delivery device 110 may include a thermoelectric generator 166, such as those described above. Thermoelectric generator 166 may be arranged on the patient's skin, optionally between housing 120 of drug delivery device 110 and the patient's skin.

As noted above, in non-limiting embodiments or aspects, drug delivery device 10, 110 may include one or more indicators 90, 190, such as a visual, audible, and/or tactile indicator. In non-limiting embodiments or aspects, thermoelectric generator 66, 166 may provide power to the one or more indicators. In non-limiting embodiments or aspects, the visual indicators are one or more light emitting diodes (LED). The LED may be a multi-color LED. In non-limiting embodiments or aspects, a plurality of LEDs of same or different colors may be provided. The LED may be provided on a top surface of the drug delivery device 10, 110 for easy viewing by the patient. The LED may be configured, e.g., through a microcontroller as described herein, to walk-through certain drug delivery steps and/or error messaging for the benefit of the patient by altering the color of the LED, the rapidity at which the LED flashes, or through a transition of flashing and constant LED signals. The LED may be configured to indicate errors with the device, such as improper attachment to the patient, occlusions in the drug expulsion mechanism, low battery warnings, and the like. The LED may also be configured to indicate to the patient an end-of-dose indicator. Optionally, multiple indicators, such as multiple LEDs, may be provided on opposing sides of the drug delivery device 10, 110 to optimize viewing from multiple directions.

As described briefly above, in non-limiting embodiments or aspects, drug delivery device 10, 110 may include one or more microcontrollers, sensors, and other electronics. One or more microcontrollers may also be in communication with a wireless communication module, such as, without limitation, a Wi-Fi module, a BLUETOOTH module, a near field communication (NFC) module, or other wireless communication circuitry. The wireless communication module may be configured to allow the drug delivery device 10, 110 to communicate with one or more other electronic devices, such as a smart phone, smart watch, tablet, computer, or other electronic device through a wireless connection. The wireless connection may be any Wi-Fi connection, BLUETOOTH connection, NFC connection, or other wireless connection. The wireless communication module may be configured for automatically pairing with one or more other electronic devices within the range of a wireless signal sent by the drug delivery device 10, 110. In non-limiting embodiments or aspects, thermoelectric generator 66, 166 powers the microcontroller and/or the wireless communication module.

In non-limiting embodiments or aspects, the wireless communication module is configured to provide one-way communication with one or more other electronic devices, such as a smart phone, smart watch, tablet, computer, or other electronic device. The one-way communication with one or more electronic devices may include communicating information regarding at least one property of the drug delivery device 10, 110, such as, without limitation, the delivery status of the drug delivery device 10, 110, fill volume of the drug delivery device 10, 110, whether the drug delivery device 10, 110 is paired with one or more other electronic devices, and/or the type of pharmaceutical composition being delivered to the patient.

In non-limiting embodiments or aspects, the wireless communication module may be configured to provide two-way communication with one or more other electronic devices, such as a smart phone, smart watch, tablet, computer, or other electronic device. The two-way communication with one or more electronic devices may include, without limitation, communicating information regarding at least one property of the drug delivery device 10, 110, such as, without limitation, the delivery status of the drug delivery device 10, 110, fill volume of the drug delivery device 10, 110, whether the drug delivery device 10, 110 is paired with one or more other electronic devices, and/or the type of pharmaceutical composition being delivered to the patient, and receiving information from the one or more electronic devices. In non-limiting embodiments or aspects, receiving information from one or more electronic devices may include control signals for controlling at least one aspect of the drug delivery device 10, 110, such as the delivery status of the drug delivery device 10, 110.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A wearable drug delivery device comprising:
a housing comprising:
a skin-facing surface defining a cannula opening; and
a viewing window;
a container disposed within the housing and configured to store a pharmaceutical composition therein;
a cannula in fluid communication with the container, the cannula transitionable from an initial position in which the cannula is disposed within the housing to a use position in which the cannula extends through the cannula opening;
a visual identifier aligned with a portion of the viewing window, wherein a position of the visual identifier relative to the viewing window is dependent on a volume of the pharmaceutical composition within the container; and
a thermoelectric generator configured to supply power to the drug delivery device,
wherein the housing is configured to be releasably attached to a patient's skin.

2. The wearable drug delivery device of claim 1, wherein the container is a flexible container.

3. The wearable drug delivery device of claim 1, wherein the container is a syringe barrel.

4. The wearable drug delivery device of claim 2 or claim 3, wherein the delivery device further includes at least one of an indicator capable of communicating a condition of the delivery device to a user and a communication module configured to transmit data relating to a status of at least one property of the delivery device.

5. The wearable drug delivery device of claim 4, wherein the thermoelectric generator is configured to supply power to the at least one indicator and/or the communication module.

6. The wearable drug delivery device of claim 4, wherein the at least one indicator is an LED.

7. The wearable drug delivery device of claim 1, wherein the thermoelectric generator is configured to be arranged between the skin-facing surface of the drug delivery device and the patient's skin.

8. The wearable drug delivery device of claim 1, wherein the thermoelectric generator comprises a plurality of thermocouples arranged on a substrate.

9. The wearable drug delivery device of claim 8, wherein the plurality of thermocouples are arranged laterally on the substrate.

10. The wearable drug delivery device of claim 8, wherein the plurality of thermocouples are arranged vertically on the substrate.

11. The wearable drug delivery device of claim 8, wherein one or more of the thermocouples comprise one or more polymers.

12. The wearable drug delivery device of claim 11, wherein the one or more polymers comprise one or more of poly(3,4-ethylenedioxythiophene) polystyrene sulfonate and/or nickel ethenetetrathiolate.

13. The wearable drug delivery device of claim 8, wherein one or more of the thermocouples comprise silicon, gold, silver, nickel, palladium, copper, antinomy, tellurium, bismuth, selenium, magnesium, and/or mixtures and alloys thereof.

14. The wearable drug delivery device of any one of claims 8-13, wherein the substrate comprises one or more of silicon, alumina, polyimide, cellulose, ceramic, fabric, and/or paper.

15. The wearable drug delivery device of claim 1, wherein the device further comprises a motor configured to expel the pharmaceutical composition from the container, and wherein the motor is powered by the thermoelectric generator.
